# EUROPEAN PATENT SPECIFICATION

(11) **EP 2 448 501 B1**
(45) Date of publication and mention of the grant of the patent: **05.08.2020**
(21) Application number: 10729765.7
(22) Date of filing: 29.06.2010
(51) Int. Cl.: A61B 17/32

(54) **ULTRASONIC DEVICE FOR CUTTING AND COAGULATING**
SCHNEID- UND GERINNUNGSULTRASCHALLGERÄT
DISPOSITIF ULTRASONIQUE POUR COUPER ET COAGULER

(30) Priority: 30.06.2009 US 221600 P; 25.06.2010 US 823231
(43) Date of publication of application: 09.05.2012
(73) Proprietor: Ethicon Endo-Surgery, Inc., Cincinnati, OH 45242 (US)
(72) Inventor: DANNAHER, William, D., Cincinnati, OH 45236 (US); BERTKE, Brian, D., Fort Thomas, KY 41075 (US); MORAN, Kenneth, Loveland, OH 45140 (US); ZINGMAN, Aron, O., Cincinnati, OH 45206 (US); REED, Matthew, L., Jamestown, OH 45335 (US); PUMMIL, JR., Larry, A., Germantown, OH 45327 (US); WEAVER, Robert, D., Fairborn, OH 45324 (US); FRAZIER, John, S., Kettering, OH 45409 (US)
(74) Representative: Tunstall, Christopher Stephen
(86) International application number: PCT/US2010/040451
(87) International publication number: WO 2011/002797

(56) References cited:
- FR-A1- 2 605 545
- US-A1- 2004 193 199
- US-A1- 2007 282 333
- US-A1- 2009 099 582

## Description

### Field of the Invention

The present invention generally relates to ultrasonic surgical systems and, more particularly, to an ultrasonic device that is optimized to allow surgeons to perform cutting, coagulation, and fine dissection required in fine and delicate surgical procedures such as an auxiliary node dissection or procedures deep within the internal body cavity.

### Background of the Invention

Ultrasonic surgical instruments are finding increasingly widespread applications in surgical procedures by virtue of the unique performance characteristics of such instruments. Depending upon specific instrument configurations and operational parameters, ultrasonic surgical instruments can provide substantially simultaneous cutting of tissue and homeostasis by coagulation, desirably minimizing patient trauma. The cutting action is typically effected by an end-effector at the distal end of the instrument, which transmits ultrasonic energy to tissue brought into contact with the end-effector. Ultrasonic instruments of this nature can be configured for open surgical use, laparoscopic or endoscopic surgical procedures including robotic-assisted procedures.

Ultrasonic surgical instruments have been developed that include a clamp mechanism to press tissue against the blade of the end-effector in order to couple ultrasonic energy to the tissue of a patient. Such an arrangement (sometimes referred to as a clamp coagulator shears or an ultrasonic transcctor) is disclosed in U.S. Pat. Nos. 5,322,055; 5,873,873 and 6,325,811. The surgeon activates the clamp arm to press the clamp pad against the blade by squeezing on the handgrip or handle. Such arrangements disclose a "tube-within-a-tube" configuration for activating the clamp arm. A challenge for a forceps-type instrument is how to pivot a clamp pad against the blade. The unique physical properties of an ultrasonic waveguide provide mechanical challenges that limit the locations where a pivot pin may be placed and how a pivot pin may interface with an ultrasonic waveguide in such a design that is truly feasible for medical procedures. Therefore, there is a need to overcome deficiencies of current instruments and prior art.

Some current designs of clamp coagulator shears utilize a foot pedal to energize the surgical instrument. The surgeon operates the foot pedal while simultaneously applying pressure to the handle to press tissue between the jaw and blade to activate a generator that provides energy that is transmitted to the cutting blade for cutting and coagulating tissue. Key drawbacks with this type of instrument activation include the loss of focus on the surgical field while the surgeon searches for the foot pedal, the foot pedal getting in the way of the surgeon's movement during a procedure and surgeon leg fatigue during long cases.

Some current designs of clamp coagulator shears utilize handles that are either of a pistol or scissors grips design. The scissor grip designs may have one thumb or finger grip that is immovable and fixed to the housing and one movable thumb or finger grip. This type of grip may not be entirely familiar to surgeons who use other open-type surgical instruments, such as hemostats, where both thumb and finger grips move in opposition to one another. Current designs have scissor arms that rotate around a fixed pivot or rotation point that is perpendicular to the longitudinal axis of the working element. This approach is limited since the relative motion between the two arms is completely rotational. This feature limits the ability to control the pressure profile between the two working ends when fully closed. Further, current designs do not allow for the user to vary the pressure profile at the working end of the instrument.

Some current designs of clamp coagulator shears are not specifically designed for delicate procedures where precise dissection, cutting and coagulation are required to avoid critical blood vessels and nerve bundles.

It would be desirable to provide an ultrasonic surgical instrument that overcomes some of the deficiencies of current instruments. The ultrasonic surgical instrument described herein overcomes those deficiencies.

US 2009/0099582 A1 describes an ultrasonic device for cutting and coagulating. The assembly includes a first shroud and a second shroud.

### Brief Description of the Figures

The novel features of the invention are set forth with particularity in the appended claims. The invention itself, however, both as to organization and methods of operation, may best be understood by reference to the following description, taken in conjunction with the accompanying drawings in which:
FIG. 1 is a perspective view illustrating an embodiment of an ultrasonic surgical instrument in accordance with the present invention;
FIG. 2 is an exploded assembly view of Fig. 1
FIG. 3 is a partial view of an alternate expression of the embodiment of Fig. 1;
FIG. 4a-b are exploded views of the ball-bearing pivot mechanism of Fig. 1;
FIG. 4c is an exploded cut-away view of the ball-bearing pivot mechanism;
FIG. 5a-b are exploded views of an first alternate expression of a pivot mechanism;
FIG. 6a-b are exploded views of an second alternate expression of a pivot mechanism;
FIG. 7a-b are exploded views of a third alternate expression of a pivot mechanism;
FIG. 8a-b are exploded views of a fourth alternate expression of a pivot mechanism;
FIGS. 9a-c are exploded views of a fifth alternate expression of a pivot mechanism;
FIGS. 10a-d are exploded views of a sixth alternate expression of a pivot mechanism;
FIGS. 11a-c are exploded views of a seventh alternate expression of a pivot mechanism;
FIGS. 12a-e are exploded assembly views of an eighth alternate expression of a pivot mechanism;
FIG. 13a is a partial view of an embodiment of the invention showing the jaw members in a closed position;
FIG. 13b is a partial view of the embodiment of Fig. 13a showing the jaw members in an opened position and a tissue stop;
FIGS. 14a-b are partial views of an alternate expression of a tissue stop shown with the jaws open and close;
FIGS. 15a-b are partial views of a second alternate expression of a tissue stop shown with the jaws open and close;
FIG. 16a-b is a perspective and elevation view showing an expression of the lever arm with a mechanism for varying jaw pressure;
FIGS. 17a-b are elevation and cut-away views of an alternate expression of the lever arm with an mechanism for varying jaw pressure;
FIG. 17c is a partial cut-away view of the lever arm showing an alternate expression of the lever arm with a mechanism for varying jaw pressure;
FIGS. 18a-c are alternate views of a torque wrench and adaptor for use with the embodiment of FIG. 1;
FIGS. 19a-d are embodiments of a torque-assist for connecting the handpiece to the ultrasonic instrument; and

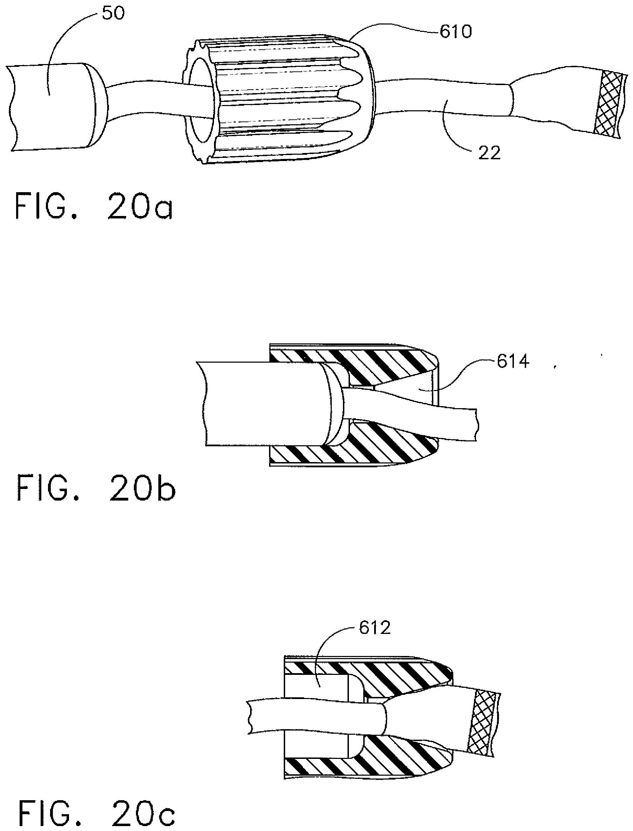

FIGS. 20a-c are perspective and cut-away views of an alternate embodiment of a torque-assist for connecting the handpiece to the ultrasonic instrument.

### Detailed Description of the Invention

Before explaining the present invention in detail, it should be noted that the invention is not limited in its application or use to the details of construction and arrangement of parts illustrated in the accompanying drawings and description. The illustrative embodiments of the invention may be implemented or incorporated in other embodiments, variations and modifications, and may be practiced or carried out in various ways. Further, unless otherwise indicated, the terms and expressions employed herein have been chosen for the purpose of describing the illustrative embodiments of the present invention for the convenience of the reader and are not for the purpose of limiting the invention.

Further, it is understood that any one or more of the following-described embodiments, expressions of embodiments, examples, etc. can be combined with any one or more of the other following-described embodiments, expressions of embodiments, examples, etc.

The present invention is particularly directed to an improved ultrasonic surgical clamp coagulator apparatus which is configured for effecting tissue cutting, coagulation, and/or clamping during surgical procedures, including delicate surgical procedures. The present apparatus is configured for use in open surgical procedures. Versatile use is facilitated by selective use of ultrasonic energy. When ultrasonic components of the apparatus are inactive, tissue can be readily gripped and manipulated, as desired, without tissue cutting or damage. When the ultrasonic components are activated, the apparatus permits tissue to be gripped for coupling with the ultrasonic energy to effect tissue coagulation, with application of increased pressure efficiently effecting tissue cutting and coagulation. If desired, ultrasonic energy can be applied to tissue without use of the clamping mechanism of the apparatus by appropriate manipulation of the ultrasonic blade.

As will become apparent from the following description, the present clamp coagulator apparatus is particularly configured for disposable use by virtue of its straightforward construction. As such, it is contemplated that the apparatus be used in association with an ultrasonic generator unit of a surgical system, whereby ultrasonic energy from the generator unit provides the desired ultrasonic actuation for the present clamp coagulator apparatus. It will be appreciated that a clamp coagulator apparatus embodying the principles of the present invention can be configured for non-disposable or multiple uses, and further configured with an integral power supply, transducer unit and controller. See, for example, U.S. Patent 6,666,875.

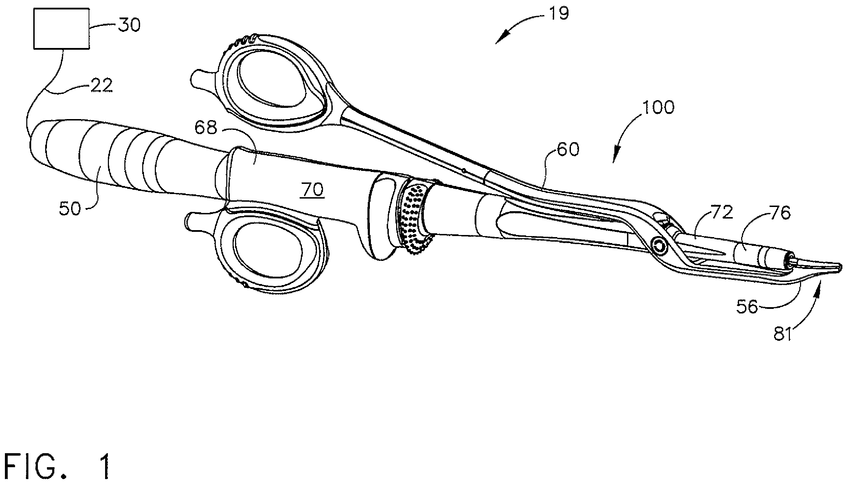

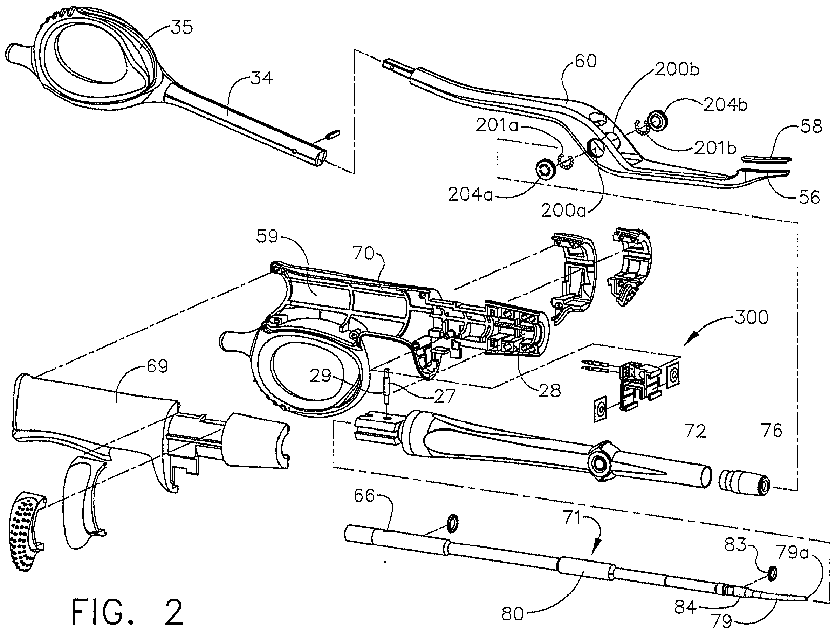

With specific reference now to Figs. 1 and 2, an embodiment of a surgical system 19, including an ultrasonic surgical instrument 100 in accordance with the present invention is illustrated. The surgical system 19 includes an ultrasonic generator 30 connected to an ultrasonic transducer 50 via cable 22, and an ultrasonic surgical instrument 100. It will be noted that, in some applications, the ultrasonic transducer 50 is referred to as a "hand piece assembly" because the surgical instrument of the surgical system 19 is configured such that a surgeon may grasp and manipulate the ultrasonic transducer 50 during various procedures and operations. A suitable generator is the GEN04 (also referred to as Generator 300) and a suitable handpiece assembly is HPBLUE, both sold by Ethicon Endo-Surgery, Inc. of Cincinnati, Ohio.

Ultrasonic transducer 50, and an ultrasonic waveguide 80 together provide an acoustic assembly of the present surgical system 19, with the acoustic assembly providing ultrasonic energy for surgical procedures when powered by generator 30. The acoustic assembly of surgical instrument 100 generally includes a first acoustic portion and a second acoustic portion. In the present embodiment, the first acoustic portion comprises the ultrasonically active portions of ultrasonic transducer 50, and the second acoustic portion comprises the ultrasonically active portions of transmission assembly 71. Further, in the present embodiment, the distal end of the first acoustic portion is operatively coupled to the proximal end of the second acoustic portion by, for example, a threaded connection.

The ultrasonic surgical instrument 100 includes a multi-piece handle assembly 68 adapted to isolate the operator from the vibrations of the acoustic assembly contained within transducer 50. The handle assembly 68 can be shaped to be held by a user in a conventional manner, but it is contemplated that the present ultrasonic surgical instrument 100 principally be grasped and manipulated in a scissor-like arrangement provided by a handle assembly of the instrument, as will be described. While multi-piece handle assembly 68 is illustrated, the handle assembly 68 may comprise a single or unitary component. The proximal end of the ultrasonic surgical instrument 100 receives and is fitted to the distal end of the ultrasonic transducer 50 by insertion of the transducer into the handle assembly 68. The ultrasonic surgical instrument 100 may be attached to and removed from the ultrasonic transducer 50 as a unit. The ultrasonic surgical instrument 100 may include a handle assembly 68, comprising mating housing portions 69 and 70 and an ultrasonic transmission assembly 71. The elongated transmission assembly 71 of the ultrasonic surgical instrument 100 extends orthogonally from the instrument handle assembly 68.

The handle assembly 68 may be constructed from a durable plastic, such as polycarbonate or a liquid crystal polymer. It is also contemplated that the handle assembly 68 may alternatively be made from a variety of materials including other plastics, ceramics or metals. Traditional unfilled thermoplastics, however, have a thermal conductivity of only about 0.20 W/m°K (Watt/ meter-°Kelvin). In order to improve heat dissipation from the instrument, the handle assembly may be constructed from heat conducting thermoplastics, such as high heat resistant resins liquid crystal polymer (LCP), Polyphenylene Sulfide (PPS), Polyetheretherketone (PEEK) and Polysulfone having thermal conductivity in the range of 20-100 W/m°K. PEEK resin is a thermoplastics filled with aluminum nitride or boron nitride, which are not electrically conductive. The thermally conductive resin helps to manage the heat within smaller instruments.

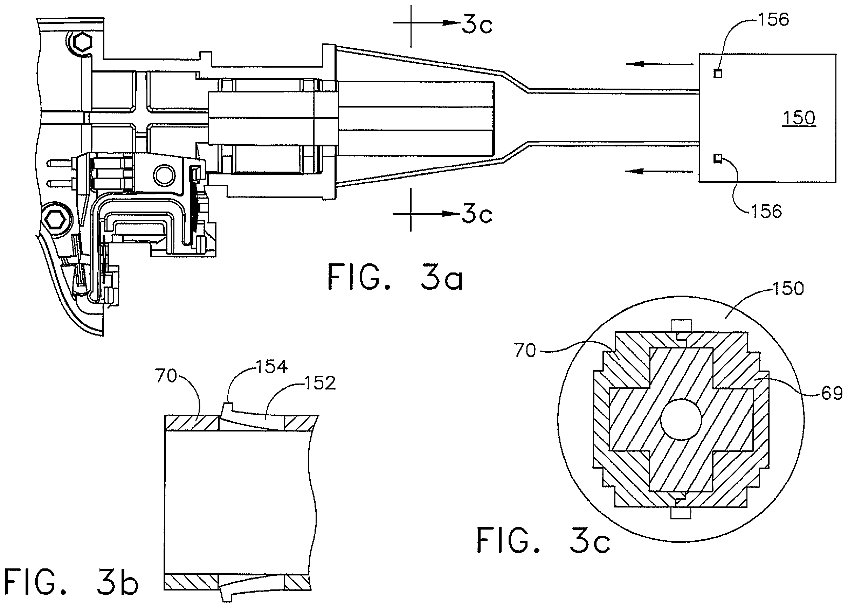

In an alternate expression shown in Figs. 3a-c, both shrouds 69 and 70, may be slimmed down or tapered at the distal tip, don't contain press pins, and have external engagement features, such as a engagement clip 152 with a protrusion 154. A third, tapered hollow cone shaped element 150 can be slid over the tip once the two shroud halves, 69 and 70, have been assembled. Cone element 150 can be made of metal or plastic and comprises apertures 156 for accepting and engaging protrusions 154. Cone element 150 may be heated and inserted over the two shroud halves, allowed to cool to provide an interference fit. Once fully assembled, the handle assembly 68 has an increased resistance to torque.

The transmission assembly 71 includes a waveguide 80 and a blade 79. It will be noted that, in some applications, the transmission assembly is sometimes referred to as a "blade assembly". The waveguide 80, which is adapted to transmit ultrasonic energy from transducer 50 to the tip of blade 79 may be flexible, semi-flexible or rigid. The waveguide 80 may also be configured to amplify the mechanical vibrations transmitted through the waveguide 80 to the blade 79 as is well known in the art. The waveguide 80 may further have features to control the gain of the longitudinal vibration along the waveguide 80 and features to tune the waveguide 80 to the resonant frequency of the system. In particular, waveguide 80 may have any suitable cross-sectional dimension. For example, the waveguide 80 may have a substantially uniform cross-section or the waveguide 80 may be tapered at various sections or may be tapered along its entire length.

Ultrasonic waveguide 80 may, for example, have a length substantially equal to an integral number of one-half system wavelengths (nλ/2). The ultrasonic waveguide 80 and blade 79 may be preferably fabricated from a solid core shaft constructed out of material, which propagates ultrasonic energy efficiently, such as titanium alloy (i.e., Ti-6A1-4V), aluminum alloys, sapphire, stainless steel or any other acoustically compatible material. Ultrasonic waveguide 80 may be fabricated into any number of lengths to address particular surgical applications, for example, thyroidectomies (short length) or conventional open procedures (long length).

Ultrasonic waveguide 80 may further include at least one radial hole or aperture 66 extending therethrough, substantially perpendicular to the longitudinal axis of the waveguide 80. The aperture 66, which may be positioned at a node, is configured to receive a connector pin 27, discussed below, which connects the waveguide 80, to the handle assembly 70.

Blade 79 may be integral with the waveguide 80 and formed as a single unit. In an alternate expression of the current embodiment, blade 79 may be connected by a threaded connection, a welded joint, or other coupling mechanisms. The distal end of the blade 79 is disposed near an anti-node 85 in order to tune the acoustic assembly to a preferred resonant frequency fₒ when the acoustic assembly is not loaded by tissue. When ultrasonic transducer 50 is energized, the distal end of blade 79 or blade tip 79a is configured to move substantially longitudinally (along the x axis) in the range of, for example, approximately 10 to 500 microns peak-to-peak, and preferably in the range of about 20 to about 200 microns at a predetermined vibrational frequency fₒ of, for example, 55,500 Hz. Blade tip 79a also preferably vibrates in the y axis at about 1 to about 10 percent of the motion in the x axis.

The blade tip 79a provides a functional asymmetry or curved portion for improved visibility at the blade tip so that a surgeon can verify that the blade 79 extends across the structure being cut or coagulated. This is especially important in verifying margins for large blood vessels. The geometry also provides for improved tissue access by more closely replicating the curvature of biological structures. Blade 79 provides a multitude of edges and surfaces, designed to provide a multitude of tissue effects: clamped coagulation, clamped cutting, grasping, back-cutting, dissection, spot coagulation, tip penetration and tip scoring.

An outer tubular member or outer shroud 72 attaches to the most distal end of handle assembly 68. Attached to the distal end of the outer shroud 72 is a distal shroud 76. Both the outer shroud 72 and distal shroud 76 may attach via a snap fit, press fit, glue or other mechanical means. Extending distally from the distal shroud 76 is the end-effector 81, which comprises the blade 79 and clamp member 56, also commonly referred to as a jaw, in combination with one or more tissue pads 58. A seal 83 may be provided at the distal-most node 84, nearest the end-effector 81, to abate passage of tissue, blood, and other material in the region between the waveguide 80 and the distal shroud 76. Seal 83 may be of any known construction, such as an o-ring or silicon overmolded at node 84.

Waveguide 80 is positioned within cavity 59 of handle assembly 68. In order to properly locate the waveguide 80 both axially and radially, pin 27 extends through opening 66 of waveguide 80 (located at a node) and engages channel 28 (formed by the mating of housing portions 69 and 70). Preferably pin 27 is made of any compatible metal, such as stainless steel or titanium or a durable plastic, such as polycarbonate or a liquid crystal polymer. In a first expression of one embodiment, pin 27 is partially coated with an elastomeric material, such as silicon, for that portion 29 of pin 27 that extends through waveguide 80 and uncoated for that portion of pin 27 that engages members 69 and 70. The silicone provides insulation from the vibrating blade throughout the length of hole 66. This enables high efficiency operation whereby minimal overheating is generated and maximum ultrasonic output power is available at the blade tip for cutting and coagulation. The lack of insulation at the ends of pin 27 allows pin 27 to be held firmly within handle assembly 68 due to the lack of insulation, which would provide deformation and movement if pin 27 were completely coated with an insulating material.

A clamp arm 60 is configured for use with the present ultrasonic surgical instrument 100 for cooperative action with blade 79 and clamp member 56, located at the distal end of clamp arm 60. Clamp arm 60 may be manufactured as a single component or manufactured in sections, attached together. The clamp arm 60 is rotatably mounted to the distal end of outer shroud 72, detailed below, and connectably attaches at the distal end of thumb ring or actuation member 34. Clamp pad 58 mounts on the clamp member 56 for cooperation with blade 79, with rotational movement of the clamp arm 60 positioning the clamp pad in substantially parallel relationship to, and in contact with, blade79, thereby defining a tissue treatment region. By this construction, tissue is grasped between clamp pad 58 and blade 79. Pivotal movement of the clamp member 56 with respect to blade 79 is affected by the provision of a pair of ball bearing rotational members on the clamp arm 60 that interface with the outer shroud 72. The outer shroud 72 is grounded to handle 68 via pin 27.

### Pivot Mechanism

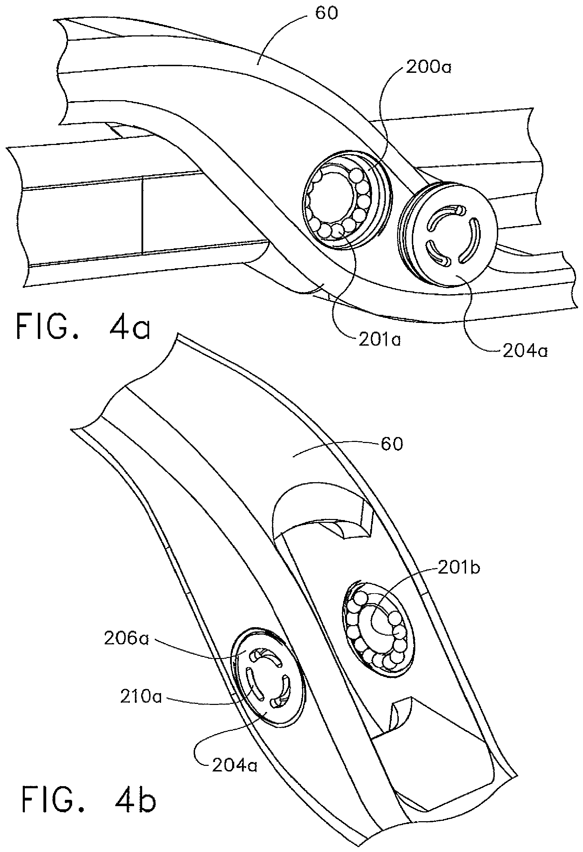

Referring now to Figs. 4a-c, in one expression, clamp arm 60 is a unitary piece that comprises two apertures 200a-b that overlap one or more bearing raceway 202a-b on either side of outer shroud 72. Bearing sets 201a-b are captured within each corresponding bearing raceway 202a-b by a bearing cap 204a-b securely inserted within a corresponding aperture 200a-b. Each cap 204a-b may be secured within aperture by glue, screw threads, laser weld or other mechanical means well known to those skilled in the art.

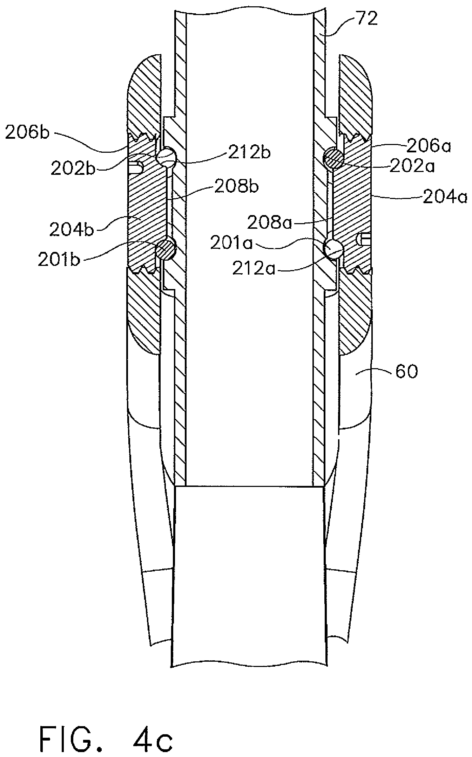

Each bearing cap 204a-b comprises a first surface 206a-b and a second surface 208a-b. First bearing surface 206a-b comprises a means 210a-b for capturing a fastening device for securing cap 204a-b within aperture 200a-b. In one embodiment, means 210a-b include one or more arcuate channels for accepting a fastening means to thread bearing cap 204a-b to aperture 200a-b (Fig. 4c). Bearing cap second surface 208a-b comprises a groove or channel 212a-b corresponding to bearing raceway 202a-b for securely capturing bearing sets 201a-b and to allow bearing sets 201a-b to travel or rotate within the channel created by bearing raceway 202a-b and channel 212a-b.

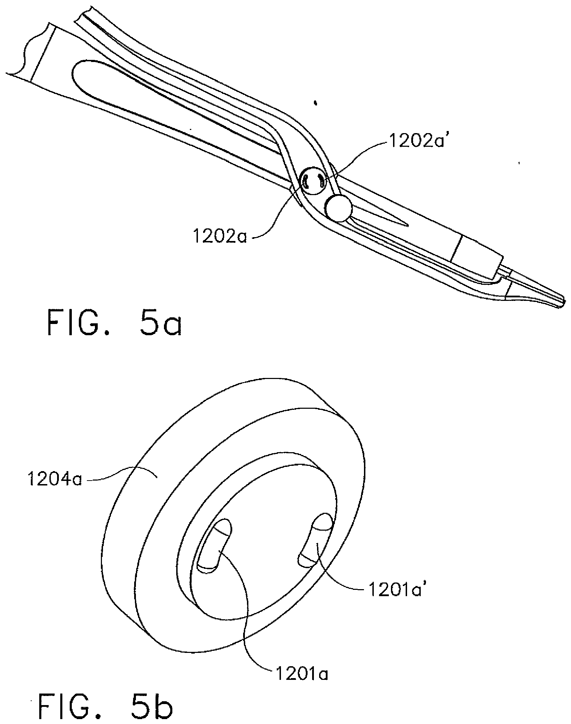

Referring now to Figs. 5a-b, an alternate expression of a bearing assembly comprises symmetrically opposed races 1202a-a' positioned on the outer shroud 72 that bearing posts 1201a-a' on bearing cap 1204a ride in on either side of the pivot. Bearing posts 1201a-a' may be machined onto cap 1204a, which may be assembled by a press fit/laser welded/glued/otherwise secured into aperture 200a-b of clamp arm 60. (Not shown, but clamp arm 60 comprises a second aperture 200b for accepting a corresponding bearing cap 1204b with bearing posts 1201b-b'.) The external geometry of the bearing cap 1204a-b may be circular or any other geometric shape in a way that the bearing cap is keyed to aid in assembly of the device.

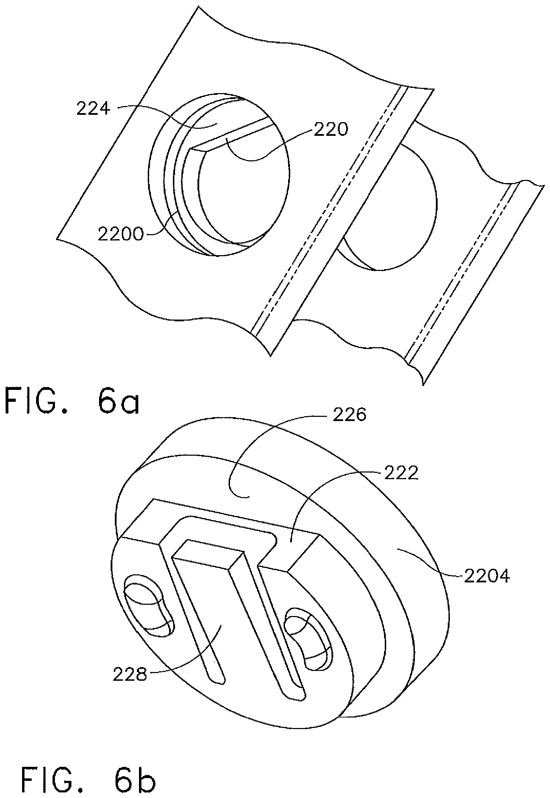

Referring now to Figs. 6a-b, in an alternate expression bearing cap 2204 includes a key feature to ensure bearing cap 2204 properly orients within aperture 2200. Key feature on bearing cap 2204, in one embodiment, is a flat surface 222 that corresponds to a flat 220 on aperture 2200. Further, surface 224 corresponds to surface 226 of bearing cap 2204 to prevent over insertion of bearing cap 2204 into aperture 2200. In addition, bearing cap 2204 may include a deflecting element 228 that is rigid or spring-like to apply a force to outer shroud 72 to remove slop in the assembly. Deflecting element 228 may be used on both bearing caps to bias the clamp arm 60 in a centered alignment with respect to the outer shroud 72 and limit the effects of tolerance stack-ups.

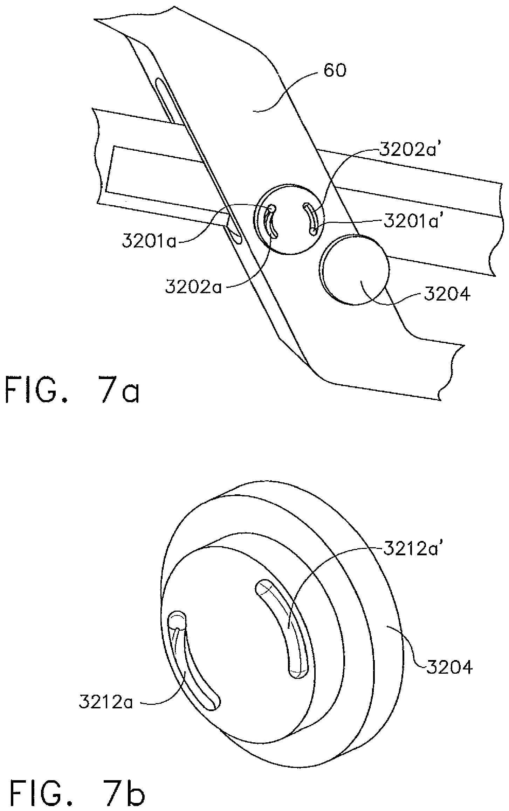

Referring now to Figs. 7a-b, in an alternate expression asymmetric raceways 3202a-a'on the outer shroud 72 may be spaced apart or partially overlap (not shown) in a concentric fashion if they are offset some distance from one another. The raceways 3202a-a' may each contain one or more ball bearings 3201a-a'. The bearings are captured by way of bearing cap 3204. Bearing cap 3204 may be mechanically fastened, press fit/laser welded/glued into aperture 3200 (not shown) of clamp arm 60. Bearing cap 3204 comprises overlapping raceways or grooves 3212a-a' to raceways 3202a-a'. Alternatively, bearing cap 3204 may have a single overlapping raceway to raceways 3202a-a'. Preferably, raceways 3212a-a' have a biasing slope or curvature such that as bearing cap 3204 is placed within aperture 3200, the biasing slope or curvature forces ball bearings 3201a-a' toward the outside of corresponding raceways 3202a-a' in order to eliminate any over tolerances of the bearing assembly.

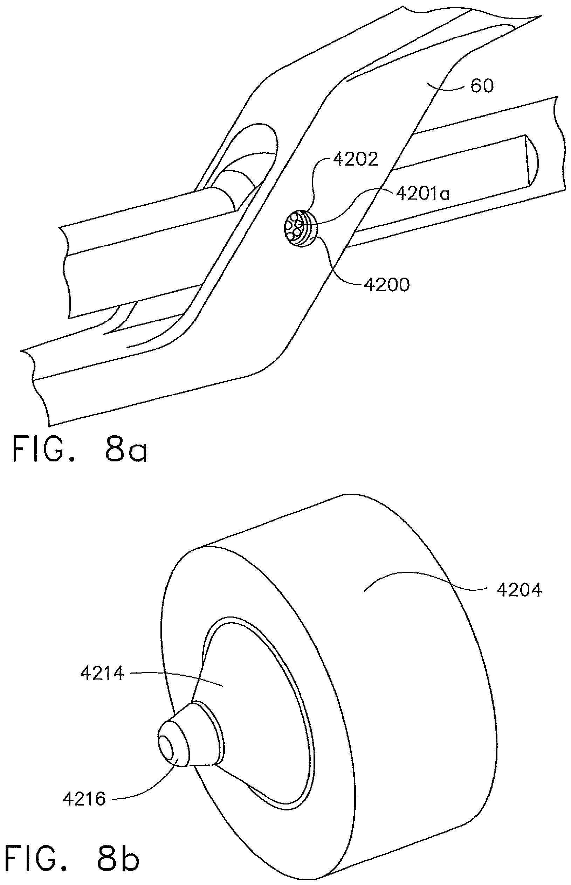

Referring now to Figs. 8a-b, in an alternate expression a cluster of small ball bearings 4201a is placed into a raceway 4202 having a circular depression on outer shroud 72. Bearing cap 4204 comprises an angled circular face 4214 that matches the geometry of the raceway 4202 and a protrusion 4216 at the distal or tapered end of circular face 4214. As bearing cap 4204 is inserted within aperture 4200 of clamp arm 60, protrusion 4216 and angled circular face 4214 force the ball bearings out from the circular depression and toward the outer perimeter of the raceway 4202 thereby adjusting for tolerances of the bearing assembly.

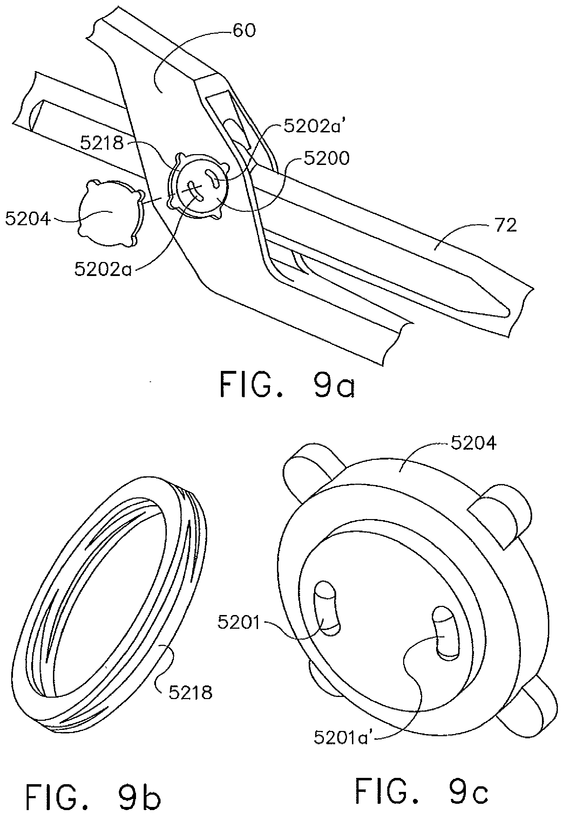

Referring now to Figs. 9a-c, in an alternate expression, a wave spring or other deflectable member 5218 is placed aperture 5200 and used in conjunction with bearing cap 5204 applies a force on outer shroud 72. Wave spring or other deflectable member 5218 may be used on both bearing caps 5204a-b to bias the clamp arm 60 in a centered alignment with respect to the outer shroud 72 and limit the effects of tolerance stack-ups.

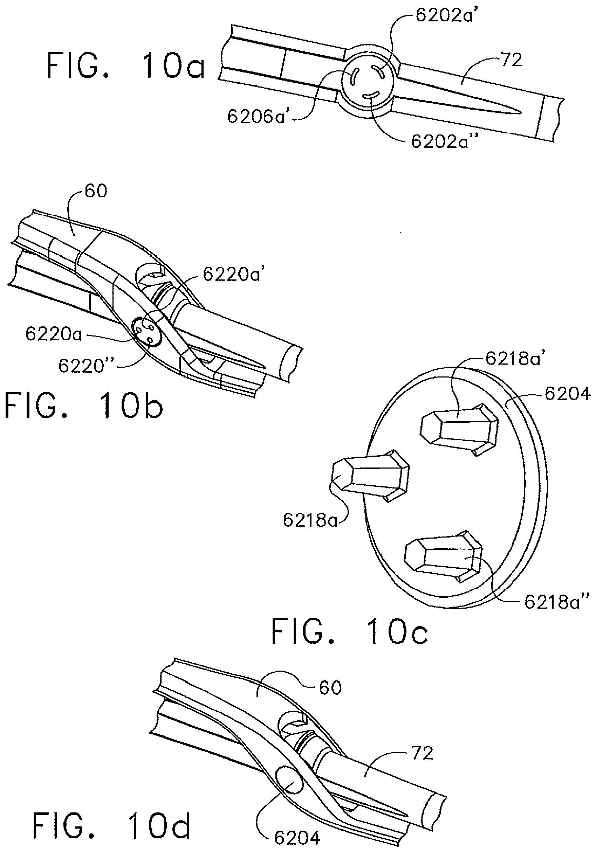

Referring now to Figs. 10a-d, in an alternate expression, asymmetric raceways 6202a-a" and 6202b-b" (not shown) (relative to other side of outer shroud 72) are situated on either side of outer shroud 72 as in the previous expressions (see, Fig. 4c). The clamp arm has at least one ball bearing- sized hole that the ball bearings may pass through. After the outer shroud 72 has been aligned with the clamp arm 60, the ball bearings are inserted into the holes and fall into the asymmetric raceways 6202a-a" and 6202b-b". One or more ball bearings may be placed in each raceway. The clamp arm may be sequentially pivoted to expose the alternate raceways to allow ball bearings to be inserted. After all of the raceways have been filled a bearing cap 6204 comprising hole plugs 6218a-a" is press fit or otherwise attached to clamp arm 60 to seal holes 6220a-a" to secure the ball bearings within raceways 6202a-a". (Not shown, but similar structure exists for the opposite site of clamp arm 60 and outer shroud 72.)

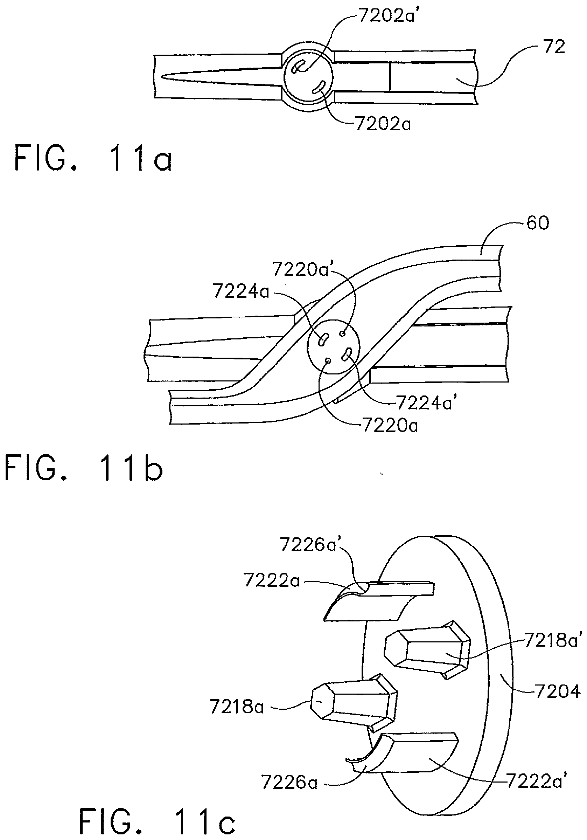

Referring now to Figs. 11a-c, in an alternate expression to the expression of Figs. 10a-d, a bearing cap 7204 comprises hole plugs 7218a-a' for use as disclosed above. Bearing cap further comprises biasing plugs 7222a-a', that when inserted into raceways 7202a-a' (via slots 7224a-a' on clamp 60) bias the ball bearings toward the outside of raceways 7202a-a'. Preferably, biasing plugs 7222a-a' have a curved recess 7226a-a' that cooperates with raceways 7202a-a' to form a channel in which the ball bearings will travel. (Not shown, but similar structure exists for the opposite site of clamp arm 60 and outer shroud 72.)

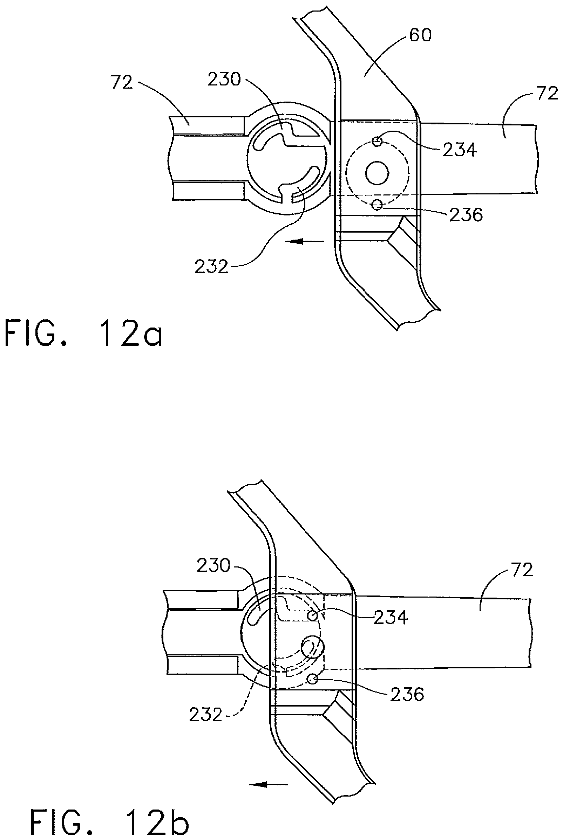

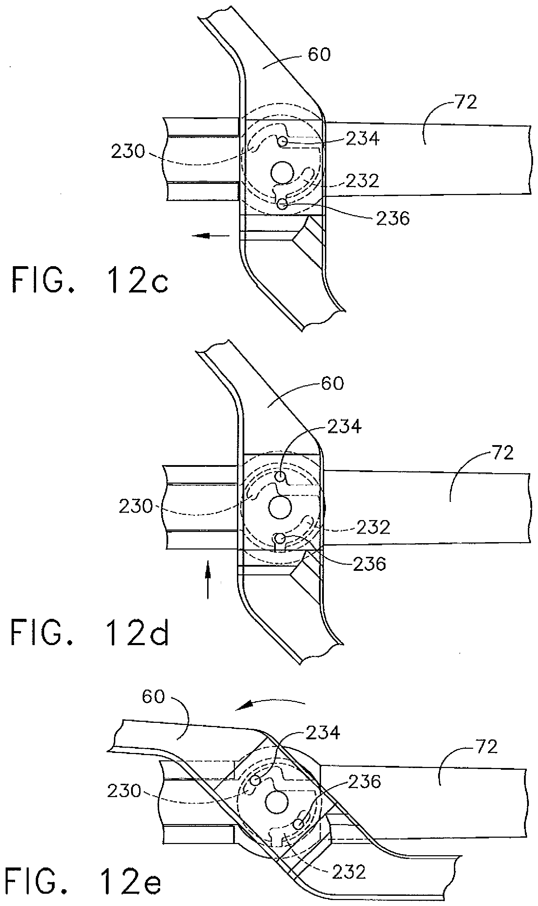

Referring now to Figs. 12a-e, an alternate expression of attaching clamp arm 60 to outer shroud 72 consists of pins 234, 236 interfacing with raceways 230 and 232. Clamp arm 60 rotates 90º relative to outer shroud 72 to insert it through the clamp arm (Fig. 12a and see Fig. 1); pivot pin 234 engages raceway 230 (Fig. 12b) and clamp arm 60 slides proximally relative to the outer shroud 72; pin 234 hits a flat in raceway 230, which corresponds to the proper location of pin 236 to engage raceway 232 (Fig. 12c); clamp arm 60 is slid upward (Fig. 12d); and clamp 60 rotates to its final position so both pins 234 and 236 engage the arcuate sections of respective raceways 230 and 232 (Fig. 12e).

### Tissue Stop

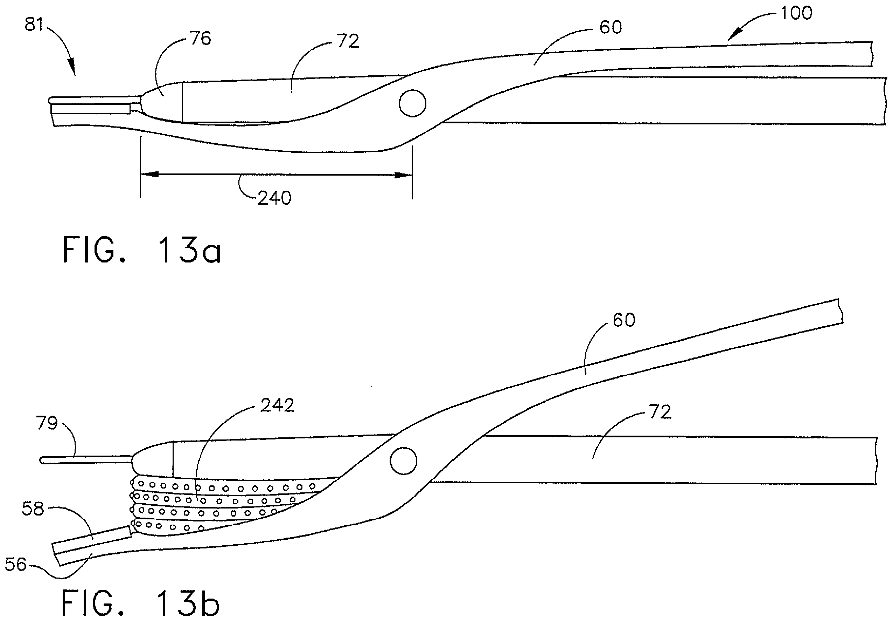

Referring now to Figs. 13a-b, tissue can get caught in the inactive area 240 between the end effector 81 and the rotation point for instrument 100. Tissue in this area 240 would not be coagulated or transected and could become damaged or cause a lowered clamp force due to obstruction. A tissue stop 242 prevents tissue from creeping outside the active area of the blade 79. Tissue stop 242 provides benefits in deep/tight access areas of internal cavities, such as the pelvis and abdomen, where a surgeon might not be able to clearly see the distal end of the instrument or might have trouble determining how much tissue is in the jaws due to the viewing angle. In a first expression baffle tissue stop 242 is positioned adjacent the outer shroud 72 and clamp arm 60. Tissue stop 242 is made of a flexible material, such as a plastic or metal that folds up like a bellows when the clamp pad 58 is closed against the blade 79. When the clamp pad 58 is disengaged from blade 79, tissue stop 242 prevents inadvertent tissue damage when the jaws are loaded with excess tissue.

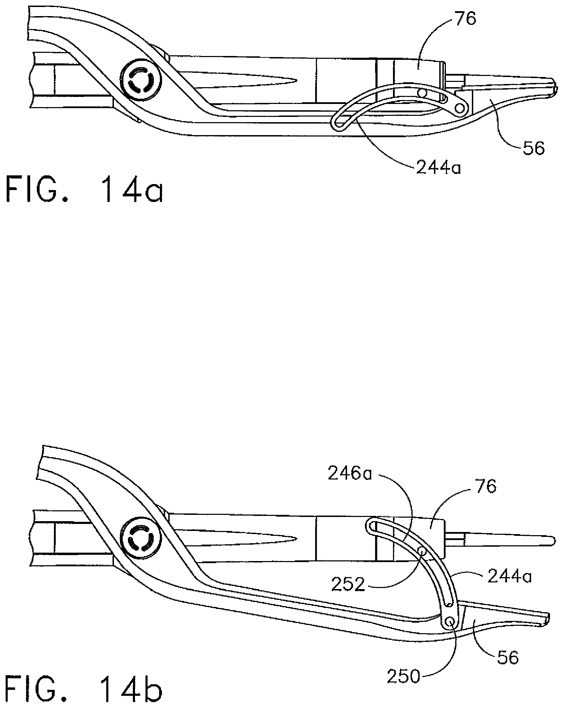

Referring to Figs. 14a-b, an alternate expression of a tissue stop includes two curved, rotating metal or plastic strips 244a-b attached on either side of distal shroud 76 and clamp member 56. One end of tissue stop 244a-b is anchored to a pin 250 at clamp member 56, pin 252 rides within slot 246a-b as clamp arm 60 rotates with respect to outer shroud 72. When the instrument is closed, the tissue stop 244a-b rest along the sides of distal shroud 76 and do not interfere with the instrument's ability to clamp over tissue. As the instrument opens, tissue stops 244a-b slide along pin 252 and rotate into a position perpendicular to blade 79. Tissue stops 244a-b would then prevent tissue from traveling proximal to pad 58.

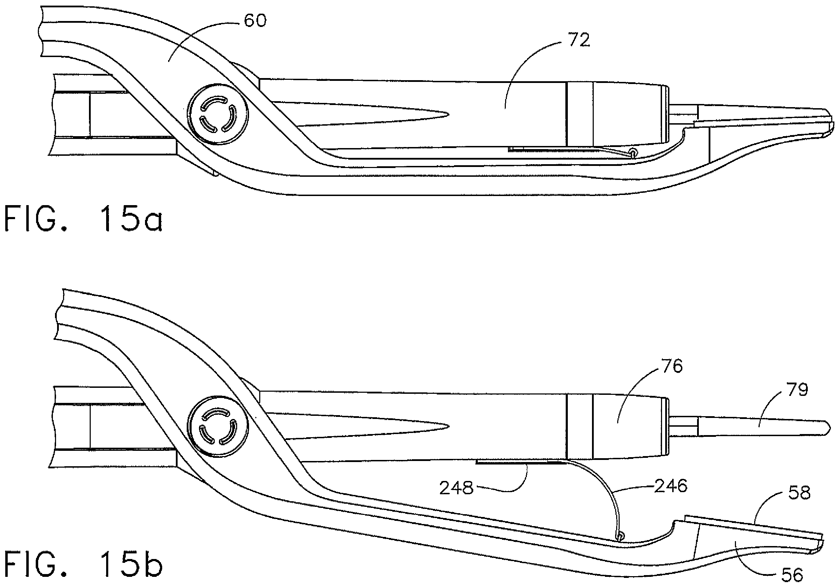

Referring to Figs. 15a-b, an alternate expression of a tissue stop utilizes a thin metal or plastic strip 246 that slides in and out of a sheath 248 located on outer shroud 72. Tissue stop 246 attaches to clamp arm 60 at a pivot point just proximal to pad 58. In one embodiment, pivot point would simply be a small bar around which one end of tissue stop 246 attaches to. When the instrument is closed, the majority of tissue stop 246 is positioned within sheath 248 and out of the way of blade 79 and pad 58. As the instrument opens, tissue stop 246 pulls out of sheath 248 to prevent tissue from traveling proximal to pad 58. As would be evident to one skilled in the art, tissue stop 246 may have tops on one end so tissue stop 246 does not slide out of sheath 248, and tissue stop 246 may be lubricated to reduce friction.

### Variable Compressive Forces

Referring again to Figs. 1 and 2, clamp arm 60 attaches to thumb ring shaft or lever 34. Thumb ring 35 attaches to the proximal end of lever 34. In one embodiment the clamp closure force is limited by the stiffness/deflection of lever 34 and the bottoming out or thumb ring 35 against housing 68.

Enhancing the ability to seal vessels can be accomplished by placing the adventitial layers of the opposing sides of a coapted vessel in direct contact with each other. Preventing this direct contact is commonly the muscular (entima) layer of the vessel. The muscular layers can be "split" within a vessel without compromising the adventitia by applying a sufficient compressive force. The muscular layers will retract enough to allow direct adventitial contact. The direct adventitial seals demonstrate higher burst pressures. In an alternate embodiment of this invention, lever 34 is constructed to include variable force control, which allows the user to create a large compressive force for muscle separation and a smaller compressive force for application of ultrasonic energy and sealing and cutting.

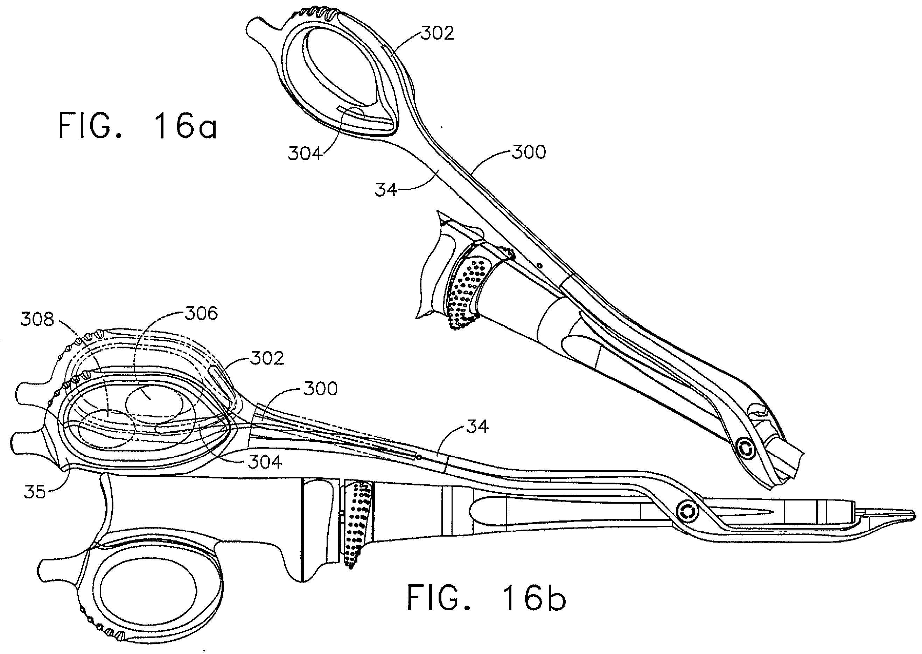

Referring to Figs. 16a-b, lever 34 further comprises a rigid spline 300, which is made from a higher bulk modulus (stiffness) material than lever 34. Rigid spline is positioned within a slot formed in lever 34 so that in one instance spline 300 does not deflect when lever 34 is depressed (not adding compressive forces at end effector) and in another instance spline 300 deflects when lever 34 is depressed (adding compressive forces at end effector). In use, if the user does not wish to add compressive forces at the end effector, the user places his/her finger at location 308, whereby spline 300 does not deflect along with lever 34. If the user wishes to add compressive forces at the end effector, the user places his/her finger at location 306, which engages prong 304 of spline 300 and causes spline 300 to deflect along with lever 34 thereby adding stiffness to lever 34, which translates to increased compressive forces at the end effector.

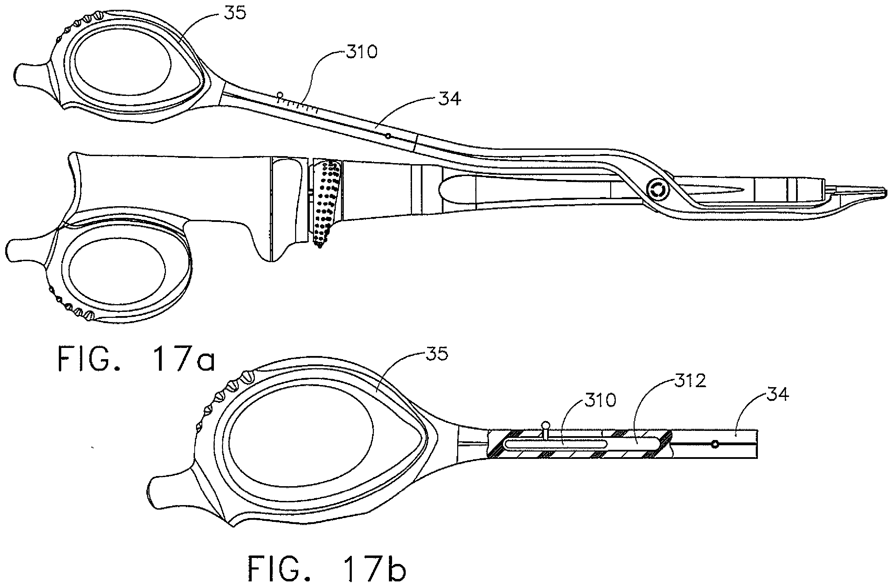

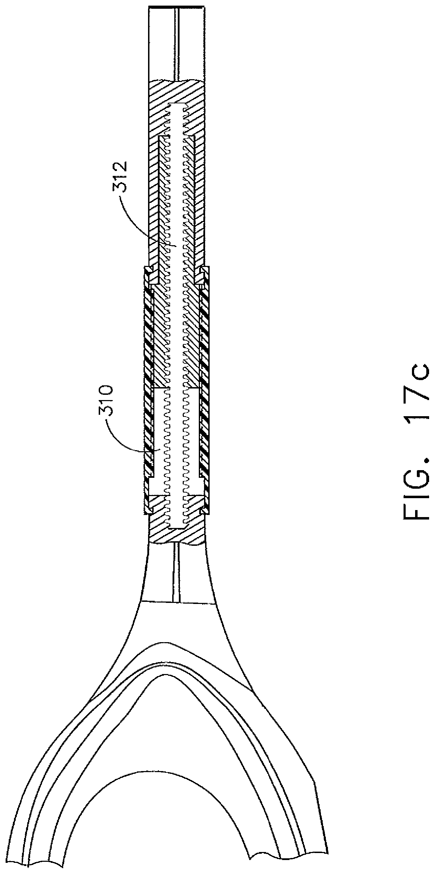

Referring to Figs. 17a-c, in an alternate expression of this embodiment, a slide bar 310 may be incorporated into lever arm 34. Slide bar 310 is made from a higher bulk modulus (stiffness) material than lever 34 and is positioned within a cavity 310 formed in lever 34. Slide bar 310 translates proximally and distally to adjust the clamp force at the end effector. As slide bar 310 moves distally, the bulk modulus of lever arm 34 decreases, and therefore, the clamp force at the end effector reduces. As slide bar 310 moves proximally, the bulk modulus of lever arm 34 increases, and therefore, the clamp force at the end effector increases. As shown in Fig. 17c, slide bar 310 may translate within lever arm 34 by means of a rotating knob connected to the slide bar by way of a series of interfacing gears. Other means of changing the bulk modulus of lever arm 34 using a higher bulk modulus material are left up to the artisan.

### Torque Wrench

Referring now to Figs. 1-2, housing 68 includes a proximal end, a distal end, and a cavity 59 extending longitudinally therein. Cavity 59 is configured to accept a switch assembly 300 and the transducer assembly 50.

In one expression of the current embodiment, the distal end of transducer 50 threadedly attaches to the proximal end of waveguide 80. The distal end of transducer 50 also interfaces with switch assembly 300 to provide the surgeon with finger-activated controls on surgical instrument 19.

Transducer 50 includes a first conductive ring 400 and a second conductive ring 410 which are securely disposed within the transducer body 50 as is described in co-pending application serial no. 11/545,784 filed on October 10, 2008, entitled MEDICAL ULTRASOUND SYSTEM AND HANDPIECE AND METHODS FOR MAKING AND TUNING.

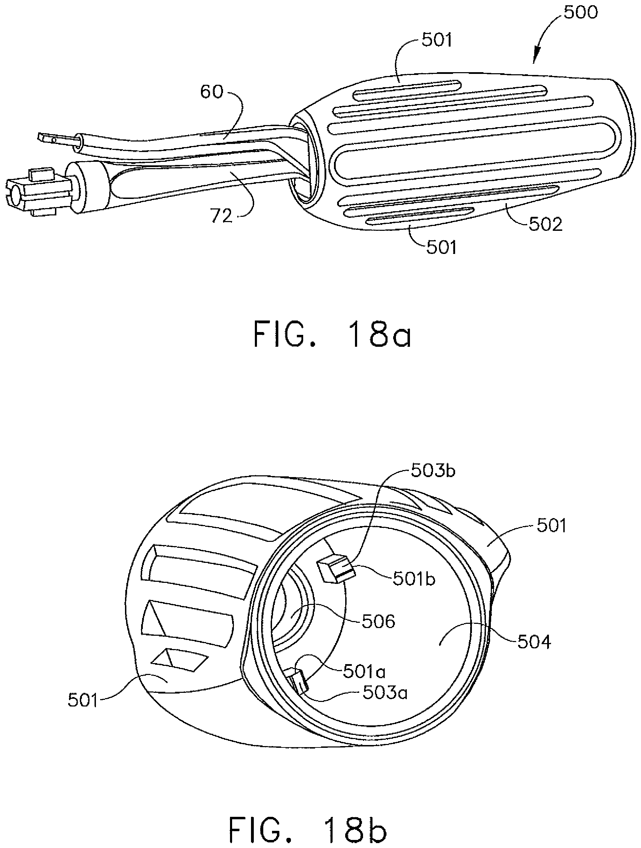

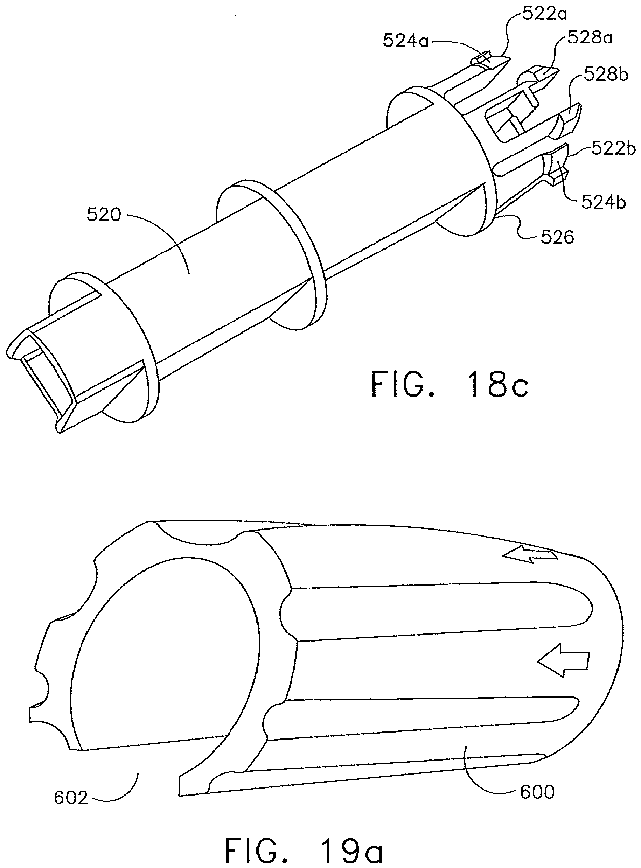

Referring now to Figs. 18a-c, a two-piece torque wrench 500 is shown. Torque wrench 500 slides over the distal end of instrument 100 to allow the user to apply the appropriate torque for attachment of transducer 50 to the proximal end of waveguide 80. The torque wrench 500, in one embodiment, includes a handgrip 502 and insert 520. Handgrip 502 is provided with teeth 501a-b (teeth 501c-d not shown) arranged about a longitudinal axis of handgrip 502. Teeth 501a-d, in one embodiment of the current invention, are disposed with a cam ramp 503a-d at a 25° angle with respect to the perpendicular angle of teeth 501a-d.

Adapter 520 includes spline gears 522a-b projecting in a perpendicular fashion from surface 526 and along the outer circumference of adapter 520. Spline gears 522a-b include cam ramps 524a-b disposed at about a 25° angle with respect to the perpendicular angle from surface 526. Other angles of the teeth and cam ramps are contemplated and left up to the designer.

In operation, torque wrench 500 is designed to maintain the torque between 4.5 in-lbs and 12 in-lbs, to be sure of correct waveguide 80 assembly to the handpiece 50 to avoid shearing the horn stud on the handpiece.

Adapter 520 is inserted into the cavity 504 of handgrip 502 so spring clips 528a-b engage a lip 506 at the distal end of handgrip 502 and is snapped into place. Preferably, the torque wrench 500 fits into a 1 inch (25.4 mm) diameter envelope, as this is the ideal diameter for most humans to grasp. Teeth 501a-d slidably engage spline gears cam ramps 524a-b. Clockwise annular motion or torque is imparted to torque wrench 500 through paddles 501. The torque is transmitted teeth 501a-d to gears 524a-b, which in turn transmit the torque to the waveguide 80 via insulated pin 27. When a user imparts 4.55-12 in-lbs. of torque and holds the handpiece 50 stationary, the ramps 503a-d and 524a-b cause the spline gears 522a-b to move or flex away from the centerline of handgrip 502 ensuring that the user does not over-tighten the waveguide 80 onto handpiece 50. When a counterclockwise torque is applied to wrench 500 via paddles 501 (and holding the handpiece 50 stationary), the user imparts a torque to the interface between the waveguide 80 and handpiece 50 in proportion to the force applied to the paddles facilitating removal of the instrument 100 from the handpiece 50. The torque wrench 500 may be constructed from a durable plastic, such as polycarbonate or a liquid crystal polymer. It is also contemplated that the wrench 500 may alternatively be made from a variety of materials including other plastics, ceramics or metals.

### Grip Assist

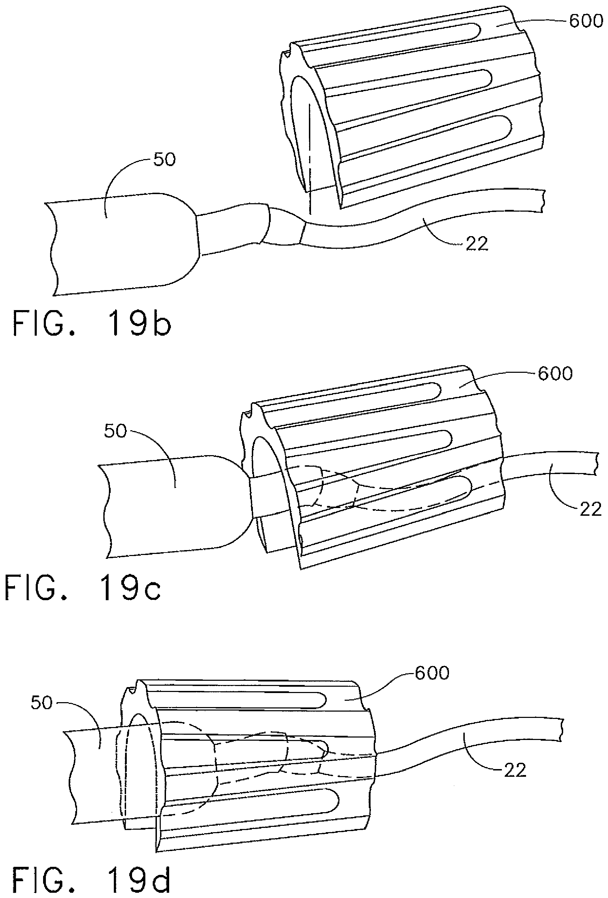

Referring now to Figs. 19a-d, a grip assist 600 increases the user's ability to resist torque while assembling and disassembling instrument 100 to handpiece 50. The grip assist 600 increases the user's ability to resist torque in two ways: it provides a larger external diameter than the handpiece 50 and it provides a greater coefficient of friction than the coefficient of friction between a surgical glove and the external coating of the handpiece.

The grip assist 600 is preferably composed of a compliant elastomer with a high coefficient of friction. This includes but is not limited to rubber, silicone, Versaflex (styrene block co-polymer), and Santoprene or it can be composed of a ridged plastic shell with the compliant elastomer over-molded to the inside of the shell. Preferably, the exterior shell or interior of the grip assist 600 may be textured to increase the coefficient of friction.

In one expression of the current invention, grip assist 600 is removable from the cable 22 by means of opening 602. Referring to Figs. 19b-d, grip assist 600 slides over cable 22 and then slides in a distal direction to sit over handpiece 50. The distal end of the grip assist frictionally attaches to the handpiece 50 proximal end while tightening the handpiece. After use, the grip assist 600 slides across the cable and may frictionally attaches to the cable plug, and out of the way of the surgeon during a procedure, but available for later use.

In an alternate expression, a grip assist 610 is reusable having a continuous outer perimeter and a first cavity 612 for mating with handpiece 50 during assembly and a second cavity 614 for mating with the cable plug and out of the way during a surgical procedure.

While the present invention has been illustrated by description of several embodiments, it is not the intention of the applicant to restrict or limit the scope of the appended claims to such detail. Numerous variations, changes, and substitutions will occur to those skilled in the art without departing from the scope of the invention. Moreover, the structure of each element associated with the present invention can be alternatively described as a means for providing the function performed by the element. Accordingly, it is intended that the invention be limited only by the scope of the appended claims.

## Claims

1. An ultrasonic surgical instrument (100) comprising:
a housing;
an outer tube (72) having a proximal end joined to the housing, the outer tube defining a longitudinal axis and at least one channel (202a-b);
an ultrasonic waveguide (80) positioned within the outer tube (72), having a proximal end, a distal end and an ultrasonically-actuated blade (79) positioned at the distal end of the waveguide (80);
an actuating lever for operating a clamp pad (58) located at the distal end of the actuating lever, the actuating lever comprises a clamp arm (60) and at least one aperture (200a-b) and operatively engages the outer tube (72) such that the at least one aperture (200a-b) is in an overlapping relationship with the at least one channel (202a-b); and
a cap (204a-b) for sealing the aperture (200a-b);
wherein movement of the actuating lever relative to the outer tube (72) positions the clamp arm (60) between open and clamped positions relative to the blade (79).

2. The ultrasonic surgical instrument (100) according to claim 1, wherein the outer tube (72) comprises opposing channels (202a-b) along the longitudinal axis.

3. The ultrasonic surgical instrument (100) according to claim 1, wherein at least one ball bearing (201a-b) is positioned within the channel.

4. The ultrasonic surgical instrument (100) according to claim 1, wherein the cap (204a-b) comprises a groove (212a-b), at least in part, in an overlapping relationship with the channel (202a-b).

5. The ultrasonic surgical instrument (100) according to claim 1, wherein the cap comprises at least one bearing post (1201a-a') in an overlapping relationship with the channel (202a-b).

6. The ultrasonic surgical instrument (100) according to claim 1, further comprising a biasing element (228).

7. The ultrasonic surgical instrument according to claim 6, wherein:
the biasing element (228) is positioned on the cap; or
the biasing element is positioned within the aperture.

8. The ultrasonic surgical instrument according to claim 6, wherein the biasing element is a spring (5219) positioned within the aperture (5200).

9. The ultrasonic surgical instrument (100) according to claim 1, wherein the at least one channel (202a-b) comprises a first channel (202a) and a second channel (202b), and the at least one aperture (200a) is in an overlapping relationship with the at least the first channel (202a).

10. The ultrasonic surgical instrument (100) according to claim 9, wherein the first channel (202a) is positioned in an opposing relationship to the second channel (202b) along the longitudinal axis.

11. The ultrasonic surgical instrument (100) according to claim 10, wherein the actuating lever comprises a second aperture (200b) in an overlapping relationship with the second channel (202b).

12. The ultrasonic surgical instrument (100) according to claim 9, wherein at least one ball bearing (201a) is positioned within at least the first channel (202a).

13. The ultrasonic surgical instrument (100) according to claim 1, wherein the cap (204a-b) comprises a groove (212a-b), at least in part, in an overlapping relationship with at least the channel (202a-b).

14. The ultrasonic surgical instrument (100) according to claim 9, wherein the cap (1204a) comprises at least one bearing post (1201a-a') in an overlapping relationship with at least the first channel (1202a-a').

15. The ultrasonic surgical instrument (100) according to claim 1, wherein the at least one channel comprises a first channel (202a) and a second channel (202b) in an opposing relationship along the longitudinal axis,
wherein the at least one aperture (200a-b) comprises a first aperture (200a) and a second aperture (200b), wherein the first aperture (200a) is in an overlapping relationship with the first channel (202a) and the second aperture (200b) is in an overlapping relationship with the second channel (202b),
wherein the cap (204a-b) comprises a first cap (204a) for sealing the first aperture (200a) and a second cap (204b) for sealing the second aperture (200b);
wherein movement of the actuating lever relative to the outer tube (72) positions the clamp arm (60) between open and clamped positions relative to the blade (79), and
wherein the ultrasonic surgical instrument (100) further comprises at least one ball bearing (201a-b) positioned within each of the first channel (202a) and the second channel (202b).

## Patentansprüche

1. Chirurgisches Ultraschallinstrument (100), umfassend:
ein Gehäuse;
eine äußere Röhre (72) mit einem proximalen Ende, das mit dem Gehäuse verbunden ist, wobei die äußere Röhre eine Längsachse und mindestens einen Kanal (202ab) definiert;
einen Ultraschallwellenleiter (80), der in der äußeren Röhre (72) positioniert ist, mit einem proximalen Ende, einem distalen Ende und einer mit Ultraschall betätigten Klinge (79), die an dem distalen Ende des Wellenleiters (80) positioniert ist;
einen Betätigungshebel zum Bedienen einer Klemmplatte (58), die sich an dem distalen Ende des Betätigungshebels befindet, wobei der Betätigungshebel einen Klemmarm (60) und mindestens eine Öffnung (200ab) umfasst und wirksam in die äußere Röhre (72) greift, sodass die mindestens eine Öffnung (200a-b) in einer überlappenden Beziehung mit dem mindestens einen Kanal (202a-b) ist; und
einen Verschluss (204a-b) zum Verschließen der Öffnung (200a-b);
wobei eine Bewegung des Betätigungshebels bezogen auf die äußere Röhre (72) den Klemmarm (60) zwischen einer offenen und einer Klemmposition bezogen auf die Klinge (79) positioniert.

2. Chirurgisches Ultraschallinstrument (100) nach Anspruch 1, wobei die äußere Röhre (72) gegenüberliegende Kanäle (202a-b) entlang der Längsachse umfasst.

3. Chirurgisches Ultraschallinstrument (100) nach Anspruch 1, wobei mindestens ein Kugellager (201a-b) in dem Kanal positioniert ist.

4. Chirurgisches Ultraschallinstrument (100) nach Anspruch 1, wobei der Verschluss (204a-b) eine Nut (212a-b) umfasst, zumindest teilweise in einer überlappenden Beziehung mit dem Kanal (202a-b).

5. Chirurgisches Ultraschallinstrument (100) nach Anspruch 1, wobei der Verschluss mindestens einen Lagerpfosten (1201a-a') in einer überlappenden Beziehung mit dem Kanal (202a-b) umfasst.

6. Chirurgisches Ultraschallinstrument (100) nach Anspruch 1, ferner umfassend ein Vorspannelement (228).

7. Chirurgisches Ultraschallinstrument nach Anspruch 6, wobei:
das Vorspannelement (228) an dem Verschluss positioniert ist; oder
das Vorspannelement in der Öffnung positioniert ist.

8. Chirurgisches Ultraschallinstrument nach Anspruch 6, wobei das Vorspannelement eine Feder (5219) ist, die in der Öffnung (5200) positioniert ist.

9. Chirurgisches Ultraschallinstrument (100) nach Anspruch 1, wobei der mindestens eine Kanal (202a-b) einen ersten Kanal (202a) und einen zweiten Kanal (202b) umfasst und die mindestens eine Öffnung (200a) in einer überlappenden Beziehung mit dem mindestens ersten Kanal (202a) ist.

10. Chirurgisches Ultraschallinstrument (100) nach Anspruch 9, wobei der erste Kanal (202a) in einer gegenüberliegenden Beziehung mit dem zweiten Kanal (202b) entlang der Längsachse positioniert ist.

11. Chirurgisches Ultraschallinstrument (100) nach Anspruch 10, wobei der Betätigungshebel eine zweite Öffnung (200b) in einer überlappenden Beziehung mit dem zweiten Kanal (202b) umfasst.

12. Chirurgisches Ultraschallinstrument (100) nach Anspruch 9, wobei mindestens ein Kugellager (201a) in mindestens dem ersten Kanal positioniert ist (202a).

13. Chirurgisches Ultraschallinstrument (100) nach Anspruch 1, wobei der Verschluss (204a-b) eine Nut (212a-b) umfasst, zumindest teilweise in einer überlappenden Beziehung mit mindestens dem Kanal (202ab) .

14. Chirurgisches Ultraschallinstrument (100) nach Anspruch 9, wobei der Verschluss (1204a) mindestens einen Lagerpfosten (1201a-a') in einer überlappenden Beziehung mit mindestens dem ersten Kanal (1202a-a') umfasst.

15. Chirurgisches Ultraschallinstrument (100) nach Anspruch 1, wobei der mindestens eine Kanal einen ersten Kanal (202a) und einen zweiten Kanal (202b) in einer gegenüberliegenden Beziehung entlang der Längsachse umfasst,
wobei die mindestens eine Öffnung (200a-b) eine erste Öffnung (200a) und eine zweite Öffnung (200b) umfasst, wobei die erste Öffnung (200a) in einer überlappenden Beziehung mit dem ersten Kanal (202a) ist und die zweite Öffnung (200b) in einer überlappenden Beziehung mit dem zweiten Kanal (202b) ist,
wobei der Verschluss (204a-b) einen ersten Verschluss (204a) zum Verschließen der ersten Öffnung (200a) und einen zweiten Verschluss (204b) zum Verschließen der zweiten Öffnung (200b) umfasst;
wobei die Bewegung des Betätigungshebels bezogen auf die äußere Röhre (72) den Klemmarm (60) zwischen einer offenen und einer Klemmposition bezogen auf die Klinge (79) positioniert, und wobei das chirurgische Ultraschallinstrument (100) ferner mindestens ein Kugellager (201a-b) umfasst, das in jedem des ersten Kanals (202a) und des zweiten Kanals (202b) positioniert ist.

## Revendications

1. Instrument chirurgical à ultrasons (100) comprenant :
un boîtier ;
un tube extérieur (72) ayant une extrémité proximale reliée au boîtier, le tube extérieur définissant un axe longitudinal et au moins un canal (202a-b) ;
un guide d'ondes ultrasonores (80) positionné à l'intérieur du tube extérieur (72), ayant une extrémité proximale, une extrémité distale et une lame actionnée par ultrasons (79) positionnée à l'extrémité distale du guide d'ondes (80) ;
un levier d'actionnement pour actionner un tampon de serrage (58) situé à l'extrémité distale du levier d'actionnement, le levier d'actionnement comprenant un bras de serrage (60) et au moins une ouverture (200a-b), et s'engageant de manière opérationnelle dans le tube extérieur (72) de telle sorte que l'au moins une ouverture (200a-b) soit en relation de chevauchement avec l'au moins un canal (202a-b) ; et
un capuchon (204a-b) pour fermer l'ouverture (200a-b) ;
le mouvement du levier d'actionnement par rapport au tube extérieur (72) positionnant le bras de serrage (60) entre des positions ouverte et serrée par rapport à la lame (79).

2. Instrument chirurgical à ultrasons (100) selon la revendication 1, le tube extérieur (72) comprenant des canaux opposés (202a-b) le long de l'axe longitudinal.

3. Instrument chirurgical à ultrasons (100) selon la revendication 1, au moins un roulement à billes (201a-b) étant positionné dans le canal.

4. Instrument chirurgical à ultrasons (100) selon la revendication 1, le capuchon (204a-b) comprenant une rainure (212a-b), au moins en partie, en relation de chevauchement avec le canal (202a-b).

5. Instrument chirurgical à ultrasons (100) selon la revendication 1, le capuchon comprenant au moins un montant de support (1201a-a') en relation de chevauchement avec le canal (202a-b).

6. Instrument chirurgical à ultrasons (100) selon la revendication 1, comprenant en outre un élément de sollicitation (228).

7. Instrument chirurgical à ultrasons selon la revendication 6,
l'élément de sollicitation (228) étant positionné sur le capuchon ; ou
l'élément de sollicitation étant positionné dans l'ouverture.

8. Instrument chirurgical à ultrasons selon la revendication 6, l'élément de sollicitation étant un ressort (5219) positionné dans l'ouverture (5200).

9. Instrument chirurgical à ultrasons (100) selon la revendication 1, l'au moins un canal (202a-b) comprenant un premier canal (202a) et un second canal (202b), et l'au moins une ouverture (200a) étant en relation de chevauchement avec l'au moins un premier canal (202a).

10. Instrument chirurgical à ultrasons (100) selon la revendication 9, le premier canal (202a) étant positionné dans une relation opposée au second canal (202b) le long de l'axe longitudinal.

11. Instrument chirurgical à ultrasons (100) selon la revendication 10, le levier d'actionnement comprenant une seconde ouverture (200b) en relation de chevauchement avec le second canal (202b).

12. Instrument chirurgical à ultrasons (100) selon la revendication 9, au moins un roulement à billes (201a) étant positionné dans au moins le premier canal (202a).

13. Instrument chirurgical à ultrasons (100) selon la revendication 1, le capuchon (204a-b) comprenant une rainure (212a-b), au moins en partie, en relation de chevauchement avec au moins le canal (202a-b).

14. Instrument chirurgical à ultrasons (100) selon la revendication 9, le capuchon (1204a) comprenant au moins un montant de support (1201a-a') en relation de chevauchement avec au moins le premier canal (1202a-a').

15. Instrument chirurgical à ultrasons (100) selon la revendication 1, l'au moins un canal comprenant un premier canal (202a) et un second canal (202b) dans une relation opposée le long de l'axe longitudinal,
l'au moins une ouverture (200a-b) comprenant une première ouverture (200a) et une seconde ouverture (200b), la première ouverture (200a) étant en relation de chevauchement avec le premier canal (202a) et la seconde ouverture (200b) étant en relation de chevauchement avec le second canal (202b),
le capuchon (204a-b) comprenant un premier capuchon (204a) pour fermer la première ouverture (200a) et un second capuchon (204b) pour fermer la seconde ouverture (200b) ;
le mouvement du levier d'actionnement par rapport au tube extérieur (72) positionnant le bras de serrage (60) entre des positions ouverte et serrée par rapport à la lame (79), et
l'instrument chirurgical à ultrasons (100) comprenant en outre au moins un roulement à billes (201a-b) positionné dans chacun du premier canal (202a) et du second canal (202b).
